(19) Europäisches Patentamt
European Patent Office
Office européen des brevets

(11) **EP 2 600 911 B1**

(12) **EUROPÄISCHE PATENTSCHRIFT**

(45) Veröffentlichungstag und Bekanntmachung des Hinweises auf die Patenterteilung:
**11.10.2017 Patentblatt 2017/41**

(21) Anmeldenummer: **11751564.3**

(22) Anmeldetag: **04.08.2011**

(51) Int Cl.:
*A61L 27/22* *(2006.01)*      *A61L 27/32* *(2006.01)*
*A61L 27/54* *(2006.01)*      *A61L 27/58* *(2006.01)*
*C01B 25/32* *(2006.01)*      *A61L 27/12* *(2006.01)*
*A61L 27/24* *(2006.01)*      *A61L 27/48* *(2006.01)*
*A61L 27/46* *(2006.01)*      *A61F 2/28* *(2006.01)*
*A61L 27/44* *(2006.01)*

(86) Internationale Anmeldenummer:
**PCT/EP2011/063470**

(87) Internationale Veröffentlichungsnummer:
**WO 2012/017045 (09.02.2012 Gazette 2012/06)**

(54) **WACHSTUMSINHIBIERTER HYDROXYLAPATIT, VERFAHREN ZU DESSEN HERSTELLUNG UND VERWENDUNG**

GROWTH-INHIBITED HYDROXYAPATITE, PROCESS FOR ITS PREPARATION AND USE

HYDROXYAPATITE À CROISSANCE INHIBÉE, PROCÉDÉ POUR SA PRÉPARATION ET UTILISATION

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **04.08.2010 DE 102010038926**

(43) Veröffentlichungstag der Anmeldung:
**12.06.2013 Patentblatt 2013/24**

(73) Patentinhaber: **Technische Universität Dresden**
**01062 Dresden (DE)**

(72) Erfinder:
• **HEINEMANN, Sascha**
**01069 Dresden (DE)**
• **HEINEMANN, Christiane**
**01069 Dresden (DE)**
• **WORCH, Hartmut**
**01187 Dresden (DE)**
• **HANKE, Thomas**
**13187 Berlin (DE)**
• **POMPE, Wolfgang**
**01337 Hartha (DE)**

(74) Vertreter: **Kailuweit & Uhlemann Patentanwälte Partnerschaft mbB**
**Bamberger Straße 49**
**01187 Dresden (DE)**

(56) Entgegenhaltungen:
**WO-A1-2007/009477      WO-A2-2006/092718**

• **JENS-HILMAR BRADT ET AL: "Biomimetic Mineralization of Collagen by Combined Fibril Assembly and Calcium Phosphate Formation", CHEMISTRY OF MATERIALS, Bd. 11, Nr. 10, 1. Oktober 1999 (1999-10-01), Seiten 2694-2701, XP55013114, ISSN: 0897-4756, DOI: 10.1021/cm991002p**

EP 2 600 911 B1

**Beschreibung**

Die Erfindung betrifft wachstumsinhibierten Hydroxylapatit (nachfolgend wHAP genannt) für die Verbesserung der Knochenheilung. Dieser unterscheidet sich von den bisher bereits eingesetzten Apatiten dadurch, dass er in physiologischen Lösungen Calcium- und Phosphationen freisetzt und diese nicht wie herkömmliche Hydroxalapatite bindet. Dadurch fördert er die Knochenneubildung und das Knochenwachstum.

Die Entwicklung neuer Biomaterialien wird hauptsächlich angetrieben durch das Bestreben, ausgereifte Gewebe- und Knochenimplantate für medizinische Anwendungen herzustellen. An diese werden jedoch je nach Implantationsort spezielle Ansprüche bezüglich der Biokompatibilität, der mechanischen Festigkeit, des Degradationsverhaltens im Falle von resorbierbaren Implantaten und der Bioaktivität gestellt. Der Begriff Bioaktivität kennzeichnet die Fähigkeit eines Materials, in Gegenwart von natürlicher oder simulierter Körperflüssigkeit die Bildung einer Apatitschicht (Knochenmineral) auf der Oberfläche zu unterstützen.

Der Säugetierknochen erfüllt im Organismus sowohl Stützfunktionen als auch metabolische Funktionen [Wintermantel, E.; Ha, S.-W.: Medizintechnik - Life Science Engineering. Springer Verlag, 2008]. Den daraus folgenden vielfältigen Anforderungen wird der Knochen durch seinen hierarchischen Aufbau und durch seine stoffliche Zusammensetzung gerecht. Die organische Knochenmatrix, die ca. 30 % des Knochens ausmacht, besteht zu ca. 95% aus Kollagen. Im Zuge eines Mineralisationsprozesses wird zwischen den Kollagenfibrillen mineralisches Calciumphosphat (vor allem Hydroxylapatit) in Gestalt von etwa zwei bis vier Nanometer dicken kristallinen Plättchen eingelagert [Kapitel Forschung aktuell. In:Max-Plank-Forschung (Hrsg.): Knochen auf den Zahn gefühlt. Bd. 1. 2005, S. 10-1]. Je nach dem Grad der Mineralisation (beim Knochen ca. 65 %) wird das flexible Grundgerüst aus Kollagen mechanisch verstärkt, wodurch sich die Festigkeit, vor allem unter Druckbelastung, enorm erhöht. Aufbauend auf derartigen Erkenntnissen wird seit einiger Zeit intensiv an Möglichkeiten zur Nachahmung natürlich vorkommender Verbundmaterialien - vor allem des Knochens - gearbeitet.

Im direkten Knochenkontakt entscheidet bei resorbierbaren Biomaterialien vor allem das Degradationsverhalten über das Schicksal der wechselwirkenden intravaskulären oder Gewebszellen. Unter dem Begriff Degradationsverhalten wird in diesem Zusammenhang die Freisetzung von Substanzen aus dem Material in das Umgebungsgewebe bzw. die Aufnahme von Substanzen aus der Umgebung und Abscheidung auf der Implantatoberfläche verstanden. Im Falle des Calcium- und Phosphathaushaltes wird die Aufnahme von Ionen aus der Umgebung unter Abscheidung auf der Implantatoberfläche in Form einer Apatitschicht als bioaktives Verhalten bezeichnet. Im Bereich der Knochenersatzmaterialien wird immer wieder berichtet, dass eine hohe Bioaktivität die Bildung einer kraftschlüssigen Verbindung zwischen Implantat und Empfängergewebe fördern kann. Andererseits berichten einige Arbeiten auch von negativen Effekten auf das Zellverhalten in Zusammenhang mit einer hohen Bioaktivität des Biomaterials. Daraus leitet sich ab, dass der vom Degradationsverhalten des Biomaterials bestimmte Ionenhaushalt, sowohl an der Implantatoberfläche als auch in den implantatnahen Gewebebereichen, entscheidenden Einfluss auf das Zellverhalten, damit auf die Knochenremodellierung und schlussendlich auf den Erfolg der Behandlung hat.

Materialien auf der Basis von Kollagen und Kollagenderivaten sind für biomedizinische Anwendungen von besonders großem Interesse. Als körpereigenes Strukturprotein kommt es ubiquitär in allen tierischen Vielzellern vor, und ist mit etwa einem Drittel der gesamten Proteinmasse deren häufigstes Protein. Es ist nahezu untoxisch, bioresorbierbar und kaum immunogen, wodurch sich eine exzellente Biokompatibilität ergibt. Als Ausgangsmaterial für industrielle Anwendungen wird meist Kollagen vom Typ I verwendet, das zum Beispiel aus Sehnen, Knorpel und Häuten von Rindern, Kälbern und Schweinen gewonnen werden kann. Mittlerweile wurden zahlreiche Produkte auf der Basis von Kollagen entwickeln und in vielen Bereichen der Kosmetik und Medizin etabliert. Bei der ebenfalls in zahlreichen Anwendungen etablierten Gelatine handelt es sich um denaturiertes Kollagen. Da allein kollagen-basierte Produkte in der Regel nicht den mechanischen Anforderungen als Knochenersatz entsprechen, werden sie mit anorganisch-nichtmetallischen Phasen substituiert oder ergänzt.

Dies sind im Bereich der Calciumphosphatbasierten Knochenersatzmaterialien vor allem Hydroxylapatit (HAP) und Tricalciumphosphat (TCP), sowie Mischungen beider Phasen eingesetzt [Tadic D, Epple M. A thorough physicochemical characterisation of 14 calcium phosphate-based bone substitution materials in comparison to natural bone. Biomaterials 2004;25 (6): 987-94]. Hydroxylapatit biologischer Herkunft (z. B. das kommerziell erhältliche Endobon, Cerabone) oder synthetischer Herkunft (z. B. Cerapatite, Ostim) in pulvriger, granulärer, blockförmiger oder pastöser Form appliziert, ist wenig bioresorbierbar [Murugan R, Ramakrishna S. Development of nanocomposites for bone grafting. Comp Sci Tech 2005;65 2385-406. Hydroxylapatit gilt aufgrund seiner Zusammensetzung, kristallinen Struktur und meist geringen Mikroporosität im neutralen pH-Bereich als unlöslich. Die vergleichbar langsam verlaufenden Abbauvorgänge in vivo werden fast ausschließlich zellulärer Resorption zugeschrieben [Detsch RA, Mayr HB, Seitz DB, Ziegler G. Is hydroxyapatite ceramic included in the bone remodelling process? An in vitro study of resorption and formation processes. Key Engineering Materials 2008;361-363 II 1123-1126]. Höhere Degradationsraten können mit HAP/TCP-Mischungen (z. B. 4-Bone, Bonesave) oder reinem TCP (z. B. Biobase, Cerasorb) erreicht werden. Das Degradationsverhalten von TCP ist jedoch nur schwer kalkulierbar.

Die Calciumphosphatphase dient im natürlichen Knochen nicht nur zur Festigkeitssteigerung, sondern ist im Zuge des Knochenstoffwechsels bzw. des Remodellings auch Calciumlieferant. Wie dieser im menschlichen Organismus im gesunden Knochen verläuft ist in groben Zügen bekannt, in welcher Weise dieser Prozess in die Defektheilung eingreift, wenn degradierbare Biomaterialien eingesetzt werden, wird im Detail noch nicht verstanden und ist Gegenstand der Forschung..Von besonderem Interesse sind Defektheilungen des osteoporotischen Knochens, die bekanntermaßen langsamer verlaufen. In einigen Arbeiten wurde gezeigt, dass die von der Löslichkeit des Implantatmaterials beeinflusste Calciumkonzentration in Implantatumgebung Auswirkungen auf das Proliferations- und Differenzierungsverhalten von Stammzellen, Osteoblasten und Osteoklasten hat [Muller P, Bulnheim U, Diener A, Luthen F, Teller M, Klinkenberg ED, Neumann HG, Nebe B, Liebold A, Steinhoff G, Rychly J. Calcium phosphate surfaces promote osteogenic differentiation of mesenchymal stem cells. J Cell Mol Med 2008;12 (1): 281-91]. Wie Detsch et al. beschreiben, verringert eine hohe Calciumionenkonzentration wie sie z. B. für TCP-basierte Materialien erhalten wird, die Resorptionsaktivität von Osteoklasten [Detsch R, Mayr H, Ziegler G. Formation of osteoclast-like cells on HA and TCP ceramics. Acta Biomater 2008;4 (1): 139-48]. Eine jüngste Veröffentlichung schreibt ungesintertem Hydroxylapatit in Form eines Komposites aus Chitosan und HAP Zytotoxizität in vitro zu, die mit einem verringerten Calcium- bzw. Magnesium-Gehalt gedeutet wird [Malafaya PB, Reis RL. Acta Biomater 2009;5 644-60].

DE 10 2004 058 893 A1 offenbart ein Verfahren zur Modifizierung von Biopolymeren. Ein Beispiel dieses Vorgehens ist der Einsatz von mit Chondroitinsulfat (CS) modifiziertem, fibrillenbildendem Kollagen, bei dem nach späterer Implantation das modifizierte Kollagen mit einem durch den metabolischen Abbau des am Kollagen befindlichen heterodimeren CS entstandenen Monomer, der Glucuronsäure, reagiert. Im Zuge dieser Reaktion werden Aminosäuren des Kollagens, vorrangig die ω-Aminogruppe des Lysins, carboxymethyliert, wodurch Regionen der Kollagenoberfläche negativ geladen werden. Als Folge der Carboxymethylierung werden in der Nachbarschaft befindliche Calciumionen gebunden. Auf diesem Weg wird eine heterogene Keimbildung von Calciumphosphatphasen eingeleitet und die Mineralbildung, z. B. die biomimetische Herstellung von HAP in der Umgebung carboxymethylierten Kollagens beschleunigt und intensiviert.

Die carboxymethylierten Template werden nach dem DCCM oder DMDM-Verfahren mineralisiert Dazu werden die carboxymethylierten Template fest an einer Oberfläche der verwendeten Membranen adsorbiert. In Gegenwart von Calcium- und Phosphationen mineralisieren die carboxymethylierten Template.

Liegt eine übersättigte Elektrolytlösung mit Ca- und $PO_4$

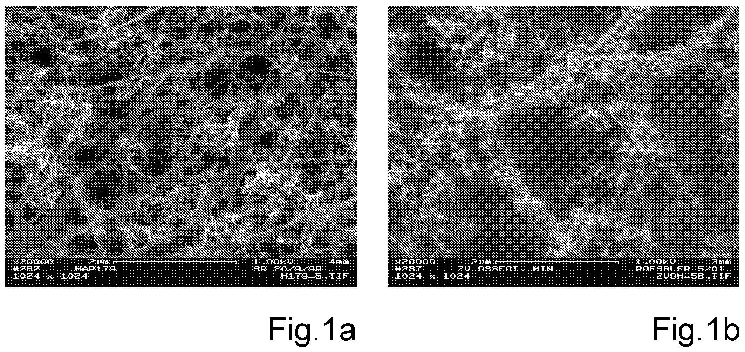

- Ionen vor, dann bilden sich auf Kollagenfibrillenoberflächen an einzelnen ausgewählten Stellen Keime aus Hydroxylapatit, die die Oberfläche nicht gleichmäßig bedecken und im Zuge des Wachstums undefiniert zusammenwachsen. Fig. 1a und Fig. 1b zeigen dazu rasterelektronenmikroskopische Aufnahmen von Hydroxylapatit, der auf Kollagen-I-Fibrillen abgeschieden wurde.

WO 2007/009477 bezieht sich auf die Herstellung von Hydroxylapatitpartikel in einer Kollagen- oder Gelatine Matrix zur Verwendung als Implantatmaterial.

**Fig. 1a** zeigt den Abscheidungsvorgang nach 15 Minuten in einer calcium- und phosphationenhaltigen gesättigten Elektrolytlösung. In der Figur sind die Kollagenfibrillen in einem verzweigten Netzwerk gut erkennbar. Auf den Oberflächen der Fibrillen hat an ausgewählten Stellen eine Mineralisation stattgefunden. Die Hydroxylaptit (HAP)- Kristallite sind durch hellere Bereiche in nadelförmiger Gestalt gut erkennbar. Die HAP-Kristallitbildung erfolgt nicht gleichmäßig (heterotaktisch) auf der Kollagenfibrillenoberfläche, sondern an einigen ausgewählten bevorzugten Stellen.

**Fig. 1b** zeigt die HAP-Bedeckung der Oberfläche nach einer Expositionszeit von zwei Stunden. Die Anzahl der Apatitkristallite hat zugenommen, sie durchdringen sich gegenseitig und bedecken aufgrund ihrer kristallographischen Struktur die darunter befindlichen Kollagenfibrillen ungleichmäßig.

Das Wachstum der HAP- Kristallite erfolgt demzufolge nicht epitaktisch in Bezug auf die Kollagenoberfläche, sondern an einzelnen ausgewählten Stellen der Oberfläche heterotaktisch mit der für Hydroxylapatit typischen Kristalltracht (Nadelbildung). Anders als im lebenden Organismus fehlt zur Keimbildung Osteocalcin, so dass die Mineralbildung nicht in Plättchenform in den Gap-Regionen der Kollagen I-Fibrillen stattfindet. Die Nadelbildung ist die Folge des Wachstums der Kristallite in kristallographischen Vorzugsrichtungen. Das Wachstum der Nadeln wird in der Vorzugsrichtung erst dann unterbrochen, wenn sie zusammenwachsen. Auch danach bleiben die gebildeten Kristallflächen wachstumsfähig. Es wachsen aufgrund der vorliegenden Oberflächenmorphologie und -energie nur andere Kristallflächen bzw. es werden neue Kristallite auf der HAP- Oberfläche gebildet. Dieser Umstand führt zur Ausbildung einer Kristalltracht des Hydroxylapatits, wie man sie von anderen bioaktiven Oberflächen gleichermaßen kennt. Diese HAP-Kristalle haben somit die unerwünschte Eigenschaft, der Elektrolytumgebung sowohl Calcium- als auch Phosphationen zu entziehen, wodurch diese wachsen und in physiologischen Lösungen Calcium- und Phosphationen gebunden werden.

Eine hohe Bioaktivität wie sie unmodifizierte Calciumphosphatphasen üblicherweise aufweisen, führt einerseits zur bisher positiv bewerteten Bildung einer Apatitschicht, aber andererseits auch zur Verarmung der Umgebung des Biomaterials an Calciumionen, was sich negativ auf die an der Remodellierung beteiligten Zellen und damit die Knochenheilung bzw. -neubildung auswirken kann. Von Bioaktivität im engeren Sinn kann demzufolge nicht gesprochen werden. Wie hoch die Calciumionenkonzentration in der Zellumgebung von Osteoblasten oder auch Osteoklasten sein muss, um ideale Voraussetzung für die Knochenneubildung zu schaffen, ist bislang noch unklar und wird in einem weiten Bereich variieren. Eindeutig zeichnet sich jedoch ab, dass Calciumphosphat-basierte Materialien benötigt werden, deren Calciumbindungs und Freisetzungsverhalten in vorbedachterweise eingestellt werden kann. Dabei sind nach dem heutigen Erkenntnisstand metastabile Calcium- Phosphat - Phasen wie z. B. Tricalciumphosphat, Bruschit und Octaclaciumphosphat ungeeignet, weil deren Auflösungsverhalten zu schnell in Bezug auf den Knochenheilungsprozess abläuft.

Aufgabe der Erfindung ist es daher, Hydroxylapatite zu formieren, deren Wachstum nach der Keimbildung inhibiert ist und die sich nach Implantation im Knochengewebe in vorbedachter Weise Calcium- und Phosphationen freisetzen. Durch diesen Effekt sollen die Proliferation und die Differenzierung knochenbildender Zellen positiv beeinflusst und die Knochenbildung gefördert werden.

Erfindungsgemäß wird die Aufgabe durch wachstumsinhibierten Hydroxylapatit gelöst, der in Agglomeraten von vorstrukturierten Kollagentemplaten enthalten ist, an denen epitaktisch Hydroxylapatitkristallite mit einer Kristallitgröße unterhalb ihres kritischen Keimradius formiert sind und der in physiologischen Elektrolytlösungen instabil ist und gelöst wird, wobei die Vorstrukturierung mit Zuckermolekülen oder nichtkollagenen Proteinen erreicht werden kann. Der Durchmesser der vorstrukturierten Kollagentemplate beträgt 1,5 - 100 nm, vorzugsweise 1,5 - 10 nm.

Der erfindungsgemäße wachstumsinhibierte Hydroxylapatit (nachfolgend wHAP genannt) liegt in Form von wHAP-enthaltenden Agglomeraten vor. Die wHAP-Agglomerate sind in der Regel kleiner als 1 $\mu$m, vorzugsweise 400 bis 600 nm groß. Die an den vorstrukturierten Kollagentemplaten aufgewachsenen wHAP-Kristallite weisen eine Kristallitgröße von 0,5 bis 20 nm, vorzugsweise von 1 bis 10 nm, besonders bevorzugt von 1 bis 5 nm auf. Im Agglomerat umschließen die vorstrukturierten Kollagentemplate mit den aufgewachsenen wHAP-Kristalliten einander bzw. sind agglomeriert. Die vorstrukturierten Kollagentemplate, an den wHAP-Kristallite epitaktisch aufgewachsen bzw. formiert sind, werden nachfolgend als Gefügebausteine bezeichnet.

Der erfindungsgemäße wachstumsinhibierte Hydroxylapatit (nachfolgend auch wHAP genannt) befindet sich in einem metastabilen Zustand, ist in physiologischen Elektrolytlösungen instabil und wird damit gelöst. Physiologische Elektrolytlösungen sind dadurch gekennzeichnet, dass die Ionenkonzentrationen (z. B. Natrium, Chlorid) und die Osmolarität der des menschlichen Serums entsprechen.

Die erfindungsgemäßen HAP-Kristallite, lösen die sich in einem schmalen Band einer Calciumkonzentration von 2,2 bis 2,7 mMol /l auf. Durch die zusätzliche Bereitstellung von Calciumionen in der Nähe eines Knochendefektes wird die Proliferation und die Differenzierung knochenbildender Zellen positiv beeinflusst und die Knochenbildung/Knochenheilung gefördert.

Weil der metastabile Zustand durch eine kinetische Hemmung des Kristallwachstums zustande kommt, wird er als wachstumsinhibierter Hydroxylapatit bezeichnet. Er unterscheidet sich von herkömmlichem durch eine dreidimensionale (räumliche), spezifische Inhibition des Wachstumsvorganges der HAP-Kristallite, wobei diese in der dritten Dimension sowohl durch ein Dickenwachstum als auch durch einen Selbstorganisationsvorgang der Gefügebausteine erreicht wird. In der zweidimensionalen Ebene wird aufgrund des spezifischen Herstellungsprozesses die Keimbildung und das Wachstum der HAP- Kristallite so gelenkt, dass ein ebenes, homogenes, nanokristallines Gefüge entsteht mit Kristallitgrößen, die sich unterhalb des kritischen Keimradius von HAP befinden.

Der Effekt der Wachstumsinhibierung wird erreicht, indem die wHAP-Kristallitgröße mithilfe des vorstrukturierten Kollagentemplats unterhalb eines kritischen Keimradius begrenzt ist.

Der kritische Keimradius r* errechnet sich bei einer vereinfachten Annahme eines kugeligen Keims zu

$$r^* = 2\gamma / \Delta g_V - \Delta g_E)$$

Hierin bedeuten $\Delta g_V$ die spezifische freie Volumenenthalpie des HAP und $\Delta g_E$ die spezifische elastische Verzerrungsenergie des HAP-Kristalliten und $\gamma$ die Grenzflächenenergie. Außerdem gilt

$$\Delta g_V = (H_S / c_S) (c_S - c)$$

Hierin bedeuten $H_S$ : die Schmelzenthalpie für HAP, $C_S$ : die Sättigungskonzentration für Ca- und Phosphationen
c : die aktuelle Konzentration

Bei epitaktischem Aufwachsen nimmt $\Delta g_E$ Werte von etwa 0,01 an und kann daher vernachlässigt werden.
Der kritische Keimradius ist demnach umso größer, je höher die Grenzflächenenergie ist. Die Grenzflächenenergie hängt einerseits von der Geometrie der Oberfläche und andererseits von der Größe der Kristallite sowie dem Anteil innerer Grenzflächen (Korngrenzen)ab. Im Falle von Nanokristallinität nimmt die Grenzflächenenergie erheblich zu, wenn die Kristallitgröße kleiner als 10 nm ist. Dieser Wert ergibt sich auch bei der Errechnung des kritischen Keimradius für Hydroxylapatit in physiologischen Lösungen.

In den Term der spezifischen freien Volumen-enthalpie $\Delta g_v$ geht $\Delta c = c_s - c$ die Übersättigung ein. Je größer die Übersättigung ist, umso kleiner wird der kritische Keimradius. Der Anteil von $\Delta g_E$ ist im Falle epitaktischen Aufwachsens auf Oberflächen von Biopolymeren sehr gering.

Kristallitgrößen des wHAP unterhalb von 10 nm werden definiert erreicht, wenn die HAP-Kristallite auf den vorstrukturierten Kollagentemplaten epitaktisch aufwachsen. Es entsteht ein Agglomerat mit einem HAP-Gefüge, das sich aufgrund der Nanokristallinität sowie der Selbstorganisation fernab vom thermodynamischen Gleichgewicht befindet.

In der dritten Dimension wird das Wachstum der HAP- Kristallite durch Agglomeratbildung der Gefügebausteine gehemmt. Die Gefügebausteine, die vorstrukturierten Kollagentemplate, an denen wHAP-Kristallite formiert sind, zeigen die überraschende Eigenschaft, sich selbst organisieren zu können. Dies ist nur dann möglich, wenn die Gefügebestandteile diffusions- bzw. migrationsfähig sind. Im Zuge dieses Prozesses lagern sich die Gefügebausteine unter Bildung von HAP/HAP- Kontakten zusammen und formieren ein sphärisches Agglomerat-Gefüge im Mikrometermaßstab, das ein weiteres Wachstum des epitaktisch aufgewachsenen Hydroxylapatits in der dritten Dimension inhibiert. Ein Optimum wird erreicht, wenn die Gefügebausteine Kugeln/ Hohlkugeln bilden. Aber auch andere sphärische Agglomerarte formieren sich je nach der Zusammensetzung der Elektrolytlösung und der Elektrolytbewegung/ -temperatur bzw. Diffusion/ Migration der beteiligten Ionen.

Die Wände dieser Hohlkugeln stellen eine Diffusionsbarriere für den Austausch von Ca- und Phosphationen dar. Wenn im Inneren einer Hohlkugel Ca- und Phosphationen durch Wachstum vorhandener HAP-Kristallite verbraucht werden, verarmt die eingeschlossene Elektrolytlösung an Ca- und Phosphationen. Im Ergebnis dieser Reaktion entstehen untersättigte Elektrolytkonzentrationen, die ein weiteres Wachstum der HAP-Kristallite zum Stillstand bringen. Demzufolge wird durch Selbstorganisation der Bausteine und der sich daraus ergebenden Architektur des Gefüges auch die Elektrolytkonzentration im Sinne einer Inhibierung des HAP-Kristallwachstums geregelt. Mit der Selbstorganisation ist damit auch eine Selbstregelung verbunden.

Zur Selbstorganisation der Makromoleküle kommt es auch dann, wenn sie nach Vorstrukturierung nur partiell mit Hydroxylapatit bedeckt sind. Neben HAP/HAP-Kontakten sind dann auch Organik/Organik sowie gemischte möglich. Je nach der Elastizität der organischen Moleküle treten unter diesen Bedingungen Abweichungen von der Kugelform auf.

Die vorstrukturierten Kollagentemplate sind dabei ausgewählt aus vorstrukturiertem, denaturiertem Kollagen, vorstrukturierten Kollagenmolekülen, wie z. B.

Tropokollagen, vorstrukturierten Kollagenmikrofibrillen, vorstrukturierten Kollagenfibrillen, vorstrukturierten Kollagenanaloga und/oder vorstrukturierten Kollagenfragmenten und/oder vorstrukturierten Kollagenderivaten, wie z. B. Gelatine.

Die durch Denaturierung und/oder die mit Zuckern oder nichtkollagenen Proteinen vorstrukturierten Kollagentemplate sind nicht länger als 1 $\mu$m, vorzugsweise 50 bis 500 nm, und in Lösungen oder Gelen diffusions- bzw. migrationsfähig. Der Durchmesser der vorstrukturierten Kollagentemplate beträgt 1,5 - 100 nm, vorzugsweise 1,5 - 10 nm. Die vorstrukturierten Kollagentemplate steuern einerseits die Keimbildung und das Keimwachstum des wHAP's sowie andererseits die Agglomeration der vorstrukturierten und mineralisierten Kollagentemplate.

Die Kollagene der vorstrukturierten Kollagentemplate können sein, rekombinantes Kollagen, Kollagen aus Eumetazoa (d. h. Gewebetiere, darunter Nesseltiere und Bilateria), Schwammkollagen der Klassen Demospongia (Hornschwämme) oder Calcarea (Kalkschwämme), ein synthetisches Kollagenanalogon, ein Kollagenderivat wie z. B. Gelatine oder eine Mischung der genannten.

Kollagenanaloga sind synthetisch hergestellte Polypeptidketten, deren Primärsequenz so gestaltet ist, dass sie bestimmte Eigenschaften der nativen Kollagenmonomere simulieren können.

Rekombinante Kollagene sind solche, deren Primärsequenz identisch ist mit der von Kollagen Typ I. Sie werden mithilfe genetisch manipulierter Mikroorganismen hergestellt und gegebenenfalls nachbehandelt.

Kollagenderivate sind Verbindungen, die sich von der Grundverbindung Kollagen formal ableiten und aus ihr herstellen lassen.

Kollagenderivate sind Verbindungen, die sich von der Grundverbindung Kollagen formal ableiten und aus ihr herstellen lassen, wie z. B. Gelatine.

Als Kollagentemplat kann jegliches denaturiertes Kollagen eingesetzt werden, d. h. teilweise oder vollständig (irreversibel) denaturiertes Kollagen und jeglichen Typs (z. B. Typ I, II, III, IV, V, VI, VII, VIII, IX, X, XI, XII, XIII, XIV, XV, XVI, XVII, XVIII, XIX, XX, XXI, XXII, XXIII, XXIV, XXV, XXVI).

Das Kollagentemplat kann sein eine Lösung oder ein Hydrogel.

Die Kollagentemplate sind soweit denaturiert oder zerkleinert, dass deren Fibrillogenese unterbunden und die Kollagentemplate in Lösung oder im Hydrogel diffusions- bzw. migrationsfähig sind. Die Länge der Kollagentemplate beträgt weniger als 1 $\mu$m vorzugsweise 50 bis 500 nm. Der Durchmesser der vorstrukturierten Kollagentemplate beträgt 1,5-100 nm, vorzugsweise 1,5-10 nm.

Die Kollagentemplate zeichnen sich durch Vorstrukturierung ihrer Oberflächen aus. Diese kann natürlicherweise vorliegen oder in vorbedachter Weise erzeugt werden. Das wird durch Zuckermoleküle bzw.

nichtkollagene Proteine erreicht. Als Zucker kommen Mono- und Disaccharide, Glycoproteine, Proteoglycane und Glykosaminoglykane in Betracht. Als nichtkollagene Proteine sind Osteocalcin, Osteonektin und Osteopontin verwendbar. Vorstrukturierung im Sinne der Erfindung heißt, dass die vorstrukturierten Kollagentemplate so funktionalisiert sind, das an deren Oberfläche Hydroxylapatit aufwachsen kann, der aufgrund der Größe, Vorstrukturierung und Diffusions- und Migrationsfähigkeit der vorstrukturierten Kollagentemplate wachstumsinhibiert wird. Die Funktionalisierung kann natürlichen Ursprungs sein oder durch Vorstrukturierung mit Zuckermolekülen oder nichtkollagenen Proteinen erreicht werden.

Nach einer vorteilhaften Ausgestaltung der Erfindung ist der wachstumsinhibierte Hydroxylapatit in Agglomeraten von Gelatinemolekülen enthalten, an denen epitaktisch HAP-Kristallite mit einer Kristallitgröße unterhalb ihres kritischen Keimradius formiert sind, wobei die Gelatinemoleküle einen Durchmesser zwischen 1,5 und 20 nm, vorzugsweise von 10 bis 15 nm und die HAP-Kristalliten, eine Kristallitgröße von 1 bis 5 nm aufweisen.

Diese Gelatinemoleküle haben eine hohe Oberflächenenergie im Vergleich zu ihrem Volumen. Aus diesem Grunde sind sie besonders geeignet für Kristallitbildungen mit vergleichbar kleiner spezifischer Volumenenthalpie. Aufgrund ihrer hohen Oberflächenenergie im Vergleich zur Volumenenthalpie sind diese Gelatinemoleküle zur Selbstorganisation in Lösungen oder Gelen fähig. Sie minimieren ihre Oberflächenenergie, indem sie sich mit anderen Gelatinemolekülen zu sphärischen Agglomeraten zusammenschließen.

Die auf diese Weise gebildeten Gefügebausteine mit einer durchschnittlichen Länge von etwa 300 nm agglomerieren zu Hohlkugeln. Diese werden als spärische wHAP-Agglomerate bezeichnet mit einer Größe kleiner als 1 $\mu$m, vorzugsweise 400 bis 600 nm.

Der erfindungsgemäß wachstumsinhibierte wachstumsinhibierte Hydroxylapatit unterscheidet sich von bisher bereits eingesetzten Apatiten dadurch, dass er in physiologischen Lösungen Calcium- und Phosphationen nicht binden, sondern freisetzen. Dadurch fördern sie in nachweisbarem Umfang die Knochenneubildung und das Knochenwachstum.

Erfindungsgemäß wird der wachstumsinhibierte Hydroxylapatit mit folgenden Schritten hergestellt:

    a) Mineralisation von vorstrukturierten Kollagentemplaten in übersättigten Ca- und Phosphationen aufweisenden Lösungen, wobei die vorstrukturierten Kollagentemplate diffusions- bzw. migrationsfähig sind, so dass eptaktisch HAP-Kristallite an den Kollagentemplaten aufwachsen und sich die mit HAP-Kristalliten aufgewachsenen Kollagentemplate aufgrund deren Diffusions- bzw. Migrationsfähigkeit agglomerieren,

    b) Abtrennen der Agglomerate,

wobei als vorstrukturierte Kollagentemplate mit Zuckermolekülen und/oder nichtkollagenen Proteinen vorstrukturierte Kollagentemplate verwendet werden.

Als vorstrukturierte Kollagentemplate werden vorstrukturiertes, denaturiertes Kollagen, vorstrukturierte Kollagenmolekülen, wie z. B. Tropokollagen, vorstrukturierte Kollagenmikrofibrillen, vorstrukturierte Kollagenfibrillen, vorstrukturierte Kollagenanaloga und/oder vorstrukturierte Kollagenfragmenten und/oder vorstrukturierte Kollagenderivate, wie z. B. Gelatine. verwendet.

Die Kollagene der vorstrukturierten Kollagentemplate können sein, rekombinantes Kollagen, Kollagen aus Eumetazoa (d. h. Gewebetiere, darunter Nesseltiere und Bilateria), Schwammkollagen der Klassen Demospongia (Hornschwämme) oder Calcarea (Kalkschwämme), ein synthetisches Kollagenanalogon, ein Kollagenderivat wie z. B. Gelatine oder eine Mischung der genannten.

Die erfindungsgemäß verwendeten vorstrukturierten Kollagentemplate sind soweit denaturiert oder zerkleinert, dass deren Fibrillogenese unterbunden aber die Kollagentemplate in Lösung oder im Hydrogel diffusions- bzw. migrationsfähig sind. Die Länge der vorstrukturierten Kollagentemplate beträgt vorzugsweise 50 bis 500 nm.

Die erfindungsgemäß verwendeten vorstrukturierten Kollagentemplate zeichnen sich durch Vorstrukturierung ihrer Oberflächen aus. Diese kann natürlicherweise vorliegen oder in vorbedachter Weise erzeugt werden. Das wird durch Zuckermoleküle bzw. nichtkollagene Proteine erreicht. Als Zucker kommen Mono- und Disaccharide, Glycoproteine, Proteoglycane und Glykosaminoglykane in Betracht. Als nichtkollagene Proteine sind Osteocalcin, Osteonektin und Osteopontin verwendbar.

Vorstrukturierung im Sinne der Erfindung heißt, dass die vorstrukturierten Kollagentemplate so funktionalisiert sind, das an deren Oberfläche Hydroxylapatit aufwachsen kann, der aufgrund der Größe, Vorstrukturierung und Diffusions- und Migrationsfähigkeit der vorstrukturierten Kollagentemplate wachstumsinhibiert wird. Die Funktionalisierung kann natürlichen Ursprungs sein oder durch Vorstrukturierung mit Zuckermolekülen oder nichtkollagenen Proteinen erreicht werden.

Die Mineralisation der vorstrukturierten Kollagentemplate erfolgt entweder stromlos in übersättigten Lösungen bei unterschiedlichem pH-Werten, die neben Calcium- und Phosphationen die vorstrukturierten Kollagentemplate in gewünschter Konzentration enthalten. Die Ionenkonzentrationen der Lösungen werden so gewählt, dass sich ein Ca/P-Verhältnis von etwa 1,67 einstellt.

Weiterhin kann die Mineralisation in Calcium- und Phosphat-Elekrolytlösungen unter Zusatz organischer Verbindungen durch ein von außen angelegtes elektrisches Feld (z. B. Potentiostat, Galvanostat) vorgenommen werden.

Möglich ist auch, die Mineralisation in Teilschritten beginnend mit einer Inkubation in einer calciumionenhaltigen Lösung vorzunehmen. Dadurch lagern sich die Calciumionen in vorbedachter Weise an den vorstrukturierten Templatoberflächen an. Erst in einem zweiten Mineralisationsschritt wird durch Inkubation des Kollagentemplats in einer phosphathaltigen Lösung die Anlagerung von HAP eingeleitet. Die zuvor im Gel gebundenen Calciumionen geben dadurch ganz maßgeblich die Wachstumsinhibierung vor. Die umgekehrte Reihenfolge ist nicht zielführend. Diese Prozesse können durch äußere elektrische Felder zusätzlich gesteuert werden.

Eine weitere vorteilhafte Mineralisiation besteht auch darin, dass die Mineralisierungsschritte räumlich getrennt werden: Die calciumhaltigen bzw. die phosphathaltigen Lösungen werden räumlich so zum Templatgel angeordnet, dass erst durch die Ionendiffusion im Gel, d. h. in Gegenwart der vorstrukturierten Template, eine Mineralisierung erfolgt. Dieser Prozess kann durch äußere elektrische Felder gesteuert werden.

Die Herstellung der wHAP-Agglomerate kann auch erfolgen, indem die Calcium- und Phosphationenhaltigen Ausgangslösungen in ein zuvor vorstrukturiertes Kollagenhydrogel - vorzugsweise bestehend aus Gelatine - diffundieren und im Gel wHAP-Kristallite gebildet werden, die sich zu wHAP-Agglomeraten zusammenlagern.

Aufgrund der Beweglichkeit der vorstrukturierten Kollagentemplate und deren Fähigkeit zur gegenseitigen Ausrichtung oder Agglomeration bilden sich nur begrenzt Calciumkeime und infolge HAP-Kristallitkeime an den Oberflächen der Kollagentemplate aus. Als Ergebnis werden wachstumsinhibierte Hydroxylapatite mit Größen von 1 bis 5 nm erhalten, die sich zu kugelförmigen Agglomeraten mit mittleren Durchmessern im zweistelligen Nanometerbereich zusammenlagern. Erfolgt die Formierung der Calciumphosphatphasen dagegen in Abwesenheit von z. B. Kollagenmolekülen oder bei einer stark gestörten Wechselwirkung mit den Kollagenmolekülen, entstehen in der Regel nadel- oder plättchenförmige Kristalle im Mikrometerbereich.

Die so gebildeten wHAP-Agglomerate können durch Entfernen des überschüssigen Templats separiert werden. Das kann beispielsweise durch Temperaturerhöhung, Säurebehandlung und Enzymbehandlung geschehen.

Nach dem erfindungsgemäßen Verfahren werden Agglomerate in Form von Pulvern/Partikeln erhalten, die superfeinkörnige, nanokristalline wHAP-Kristallite mit einer Kristallgröße von ca. 0,5- 20nm, vorzugsweise von 1- 20 nm, besonders bevorzugt von 0,5-5nm, ganz besonders bevorzugt von 1 - 5 nm enthalten.

Der wachstumsinhibierte HAP wird nach einer vorteilhaften Ausgestaltung des erfindungsgemäßen Verfahrens auf Oberflächen von Gelatinemolekülen erzeugt, die infolge des Herstellungsprozesses nicht mehr zur Fibrillogenese fähig sind. Wachstumsinhibierter HAP bildet sich auf Gelatinemolekülen, deren Durchmesser zwischen 1,5 und 20 nm, vorzugsweise zwischen 10 bis 15 nm liegen. Unter diesen Randbedingungen bilden sich auf der Moleküloberfläche Gefüge mit HAP-Kristalliten, deren mittlere lineare Korngöße nicht größer als 1 bis 5 nm ist.

Die Beschaffenheit der wHAP-Agglomerate und der wHAP-Kristallite wirkt sich auf das Lösungs-Fällungsverhalten im Kontakt mit natürlichen oder simulierten Körperflüssigkeiten aus, indem unmodifizierte, bioaktive HAP-Kristallite unter Calcium- und Phosphataufnahme wachsen, die mit Kollagentemplat bedeckten, wHAP-Kristallite hingegen keine Ionen aus der Umgebung aufnehmen und stattdessen aufgelöst werden können.

Die erfindungsgemäßen wachstumsinhibierten HAP-Kristallite sind biokompatibel.

Beim Prozess der Vorstrukturierung verändert das Kollagentemplat durch Wechselwirkung mit den oben genannten Substanzen seine Morphologie. Damit einhergehend verändern sich die Ladungsverhältnisse und damit die Primär- bis Ternärstruktur des Templates sowie die Anordnung der vorstrukturierten Templatbestandteile (Quartäre Struktur) zueinander.

Je nach Vorstrukturierung der Kollagentemplate entstehen im Mineralisierungsprozess superfeinkörnige wHAP-Kristallite mit in der Regel Größen von 1 bis 5 nm auf den Templatoberflächen. Aufgrund der gewählten Herstellungsbedingungen wird eine quasi-Einkristallbildung verhindert. In dem Bestreben, die Oberflächenenergie zu minimieren, formieren sich die wHAP-Kristallite zu wHAP-Agglomeraten (=Kompositteilchen) mit Größen von typischerweise 500 nm mit quasi-amorpher Gestalt (aus energetischen Gründen annähernd kugelförmig) und dem obengenannten Eigenschaftsprofil.

Die wachstumsinhibierten HAP-Kristallite können als Mineralkomponente in andere, vorzugsweise organische, Materialien eingearbeitet werden. Dadurch kann diesen Materialien die Eigenschaft der Calcium- und Phosphatfreisetzung verliehen werden. Außerdem können die Degradationseigenschaften der HAP-Kristallite angepasst werden. Die Festigkeit des Komposits erhöht sich mit steigendem Anteil der Nanophasen.

Auf diese Weise erhaltene Kompositmaterialien sind biomedizinisch als Implantatmaterial einsetzbar, bei dem neben Biokompatibilität, Degradabilität und hinreichender Festigkeit eine Freisetzung von Calcium- und Phosphationen gefordert wird.

Der erfindungsgemäße wachstumsinhibierte Hydroxylapatit kann zur Beschichtung von Implantatmaterialien eingesetzt werden.

## Ausführungsbeispiele

Anhand beigefügter Darstellungen und Ausführungsbeispiele wird die Erfindung näher erläutert. Dabei zeigen:

Fig.1a     TEM-Aufnahme Aufwachsen von HAP auf Kollagenfibrillen,

Fig.1b     TEM-Aufnahme ungehindertes Wachstum HAP vorstukturiertes Kollagentemplat,

Fig. 2    Höchstauflösende TEM - Aufnahme eines vorstrukturierten Kollagenmoleküls mit epitaktisch abgeschiedenen wHAP,

Fig. 3    TEM Aufnahme sphärischer wHAP-Agglomerate,

Fig. 4    Diagramm Lösungs-Fällungsverhalten von unterschiedlichem HAP.

**Ausführungsbeispiel 1:**

Lösliches Kollagen I (Vitrogen 100 (purified collagen) in 0,012 N HCl; 3 mg/ml, Cohesion, Palo Alto, USA) steht gebrauchsfertig zur Verfügung.

Low molecular weight Chitosan (Sigma) wird in 0,01 N HCl gelöst, so dass eine Stammlösungen der Konzentrationen 2 mg/ml vorliegt.

Eine Pufferlösung (1,52 mg/ml $KH_2PO_4$, 7,12 mg $Na_2HPO_4$, 0,63 mg/ml NaCl in entionisiertem Wasser) wird hergestellt.

Kollagenlösung und Chitosanlösung werden in einem Volumenverhältnis von 2:3 gemischt. Das entspricht einem Masseverhältnis von 1:1. Diese Mischung wird in einem Volumenverhältnis von 1:1 mit der Pufferlösung vermischt.

Der Ansatz reagiert bei 37 °C über einen Zeitraum von 24 h. Es werden vorstrukturierte Kollagenmoleküle erhalten. Nach Abzentrifugieren (10000 Umdrehungen/Minute, 15 Minuten, bei 21 °C) werden die vorstrukturierten Kollagenfibrillen in 50 °C warme Gelatine (10 mg/ml) eingebracht. Typische Konzentrationen des Kollagens liegen zwischen 2,5-25 m-%.

Der so hergestellte Templatansatz wird bei 4 °C in ein Gefäß eingeschichtet und geliert, dass das Gefäß in zwei etwa gleich große Bereiche geteilt wird. Im ersten Teilschritt wird das Templatgel in einer calciumionenhaltigen Lösung inkubiert. Dadurch lagern sich die Calciumionen in vorbedachter Weise an den vorstrukturierten Templatoberflächen an. Erst in einem zweiten Schritt wird durch Inkubation des Gels in einer phosphathaltigen Lösung die Mineralisierung eingeleitet. Die Ionenkonzentrationen der Lösungen werden so gewählt, dass sich ein Ca/P-Verhältnis von etwa 1,67 einstellt. Die zuvor im Gel gebundenen Calciumionen geben dadurch ganz maßgeblich die spätere Wachstumsinhibierung vor. Die umgekehrte Reihenfolge ist nicht zielführend.

In einer besonders bevorzugten Variante erfolgt eine räumliche Trennung der Mineralisierungsschritte: Die calciumhaltigen bzw. die phosphathaltigen Lösungen werden räumlich so zum Templatgel angeordnet, dass erst durch die Ionendiffusion im Gel, d. h. in Gegenwart der vorstrukturierten Template, eine Mineralisierung erfolgen kann. Dieser Prozess kann durch äußere elektrische Felder gesteuert werden.

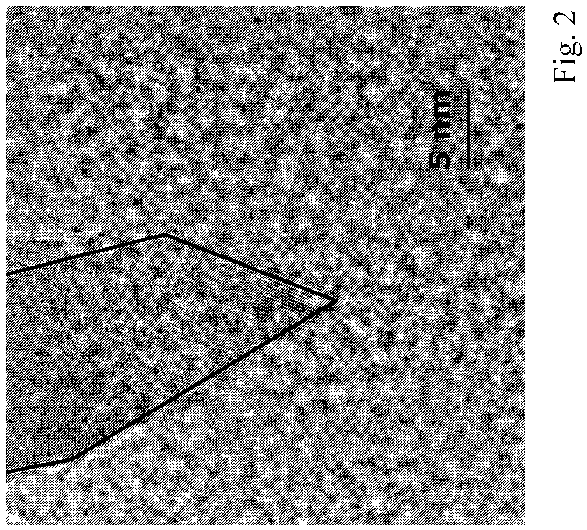

**Fig. 2** zeigt eine höchstauflösende transmissionselektronenmikroskopische Aufnahme eines vorstrukturierten Kollagenmoleküls mit epitaktisch abgeschiedenen wHAP- Kristalliten. Der Radius des Moleküls beträgt etwa 7 nm und die mittlere lineare Kristallitkorngröße liegt zwischen 1 und 5 nm. Diese Aufnahme konnte nur transmissionselektronenmikroskopisch auf einem Kohlenstoffnetz hergestellt werden. Der amorphe Kohlenstoff erscheint in der Aufnahme in verschiedenen Grautönen. Um das Kollagenmolekül in der Aufnahme deutlicher erkennbar zu machen, wurde es mit gelben Linien grob umgrenzt. Man erkennt innerhalb der Umgrenzung einzelne Bereiche, die sich durch eine parallele Streifung von Linien auszeichnen. Das sind die Netzebenen von wHAP-Kristalliten. Ob die gesamte Oberfläche mit HAP-Kristalliten bedeckt ist, lässt sich aus dieser Aufnahme nicht eindeutig zu beantwortet. Daher der Anspruch auch auf Teilbedeckung.

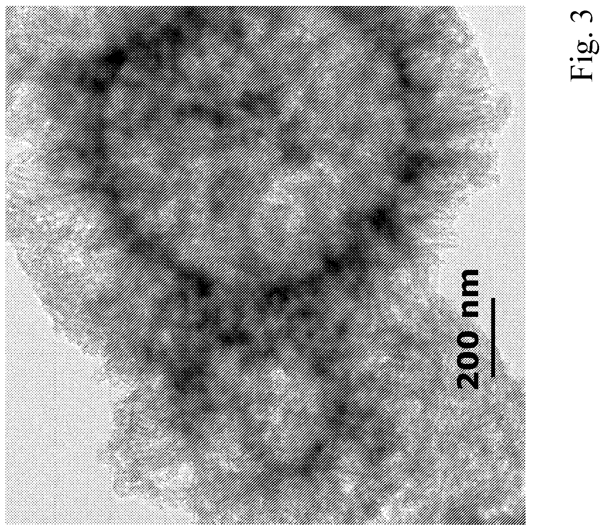

**Fig. 3** zeigt eine hochauflösende transmissionselektronenmikroskopische Aufnahme, die den Aufbau zweier spärischer wHAP-Agglomerate verdeutlich. Es ist erkennbar, dass es sich dabei um Hohlkugeln handelt. Die in Fig. 2 vergrößert dargestellten Elemente organisieren sich selbst, indem sie sich gemäß ihren Längsachsen parallel Aneinanderlagern und so die Agglomerate aufbauen.

Calcium- und Phosphationen diffundieren in allen Fällen über einen Zeitraum von bis zu 4 Wochen in das Templatgel und führen zur Mineralisierung der vorstrukturierten Template. Durch die freie dreidimensionale Beweglichkeit aller beteiligten Teilchen (Ionen und Templat) richten sich die vorstrukturierten und mineralisierten Templatmoleküle gegeneinander so aus, dass ein ungebremstes Kristallwachstum verhindert wird. Stattdessen lagern sich die wachstumsinhibierten Kompositteilchen mit dem Ziel der Minimierung der Oberflächenenergie zu Agglomeraten zusammen.

Die im Templatgel gebildeten wachstumsihibierten Kompositteilchen können durch Entfernen des überschüssigen Templats separiert werden. Das kann beispielsweise durch Temperaturerhöhung, Säurebehandlung und Enzymbehandlung geschehen. Im bevorzugten Fall werden die Kompositagglomerate (=wHAP) nach Erwärmen auf 50°C und Zentriguation für 10 Minuten bei 5000 g separiert und anschließend getrocknet.

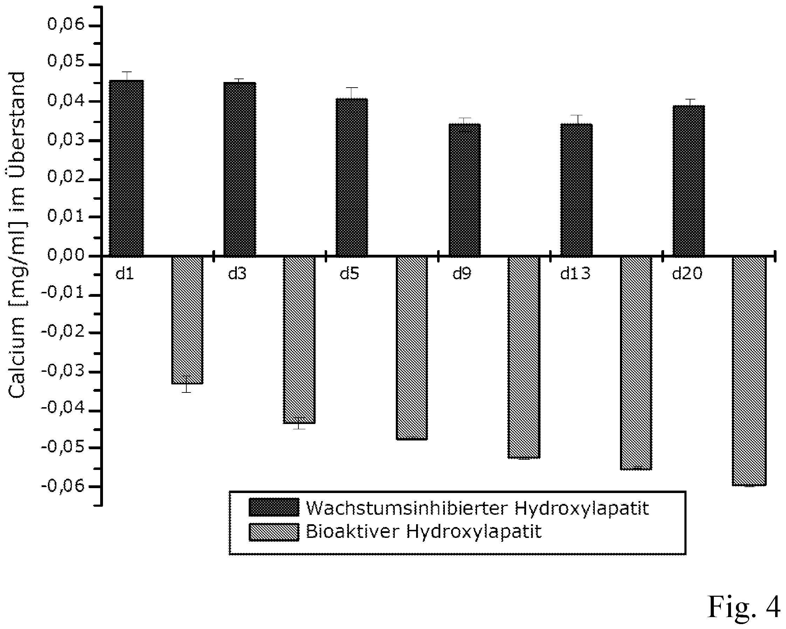

Das Lösungs-/Fällungsverhalten von konventionellem bioaktivem HAP und nach der beschriebenen Methode hergestelltem wachstumsinhibierten HAP (wHAP) wurde untersucht und im Diagramm nach **Fig. 4** dargestellt. Für dessen Erstellung wurden jeweils gleiche Massen des bioaktiven HAP und des wHAP in 2 ml einer simulierten Körperflüssigkeit bei 37 °C inkubiert und die Calciumkonzetration im Überstand über einen Zeitraum von 20 Tagen gemessen. Bei der dafür angewendeten o-Kresolphtalein-Komplex-Methode reagiert Calcium in alkalischer Lösung mit o-Kresolphtalein unter Bildung eines violetten Farbkomplexes, dessen Intensität zur Calciumkonzentration in der Probe proportional ist.

Für den Test wurden jeweils 10 μl Probenlösung in Mikrotiterplatten überführt. Anschließend wurden je 300 μl der Messreagenz zupipettiert, die durch Mischen der im Calcium-Kit enthaltenen Reagenzien 1 (AMP Puffer, pH 10,7) und 2 (o-Kresolphthalein Komplexon, 8-Hydroxyquinolin, HCl, Detergenz) im Verhältnis 1:1 erhalten. Nach 10-minütiger Durchmischung auf dem Schüttler wurde die Absorption des entstandenen Farbstoffes bei 570 nm gemessen. Eine Kalibriergerade wurde anhand einer Verdünnungsreihe des im Kit enthaltenen Calciumstandards (2,5 mM) erstellt.

Die für den bioaktiven HAP ermittelten negativen Werte kennzeichnen die Aufnahme von Calcium aus der Lösung und ein Wachsen der Mineralphase. Im Gegensatz dazu kennzeichnen die für den wHAP ermittelten positiven Werte, dass dieser Calcium in die Lösung abgibt und somit nicht wächst.

**Patentansprüche**

1. Wachstumsinhibierter Hydroxylapatit enthalten in Agglomeraten von vorstrukturierten, Kollagentemplaten, deren Fibrillogenese unterbunden ist, an denen epitaktisch Hydroxylapatitkristallite mit einer Kristallitgröße unterhalb ihres kritischen Keimradius formiert sind und der in physiologischen Elektrolytlösungen instabil ist und gelöst wird, wobei die Vorstrukturierung mit Zuckermolekülen oder nichtkollagenen Proteinen erreicht werden kann.

2. Wachstumsinhibierter Hydroxylapatit nach Anspruch 1, **dadurch gekennzeichnet, dass** die Agglomerate kleiner als 1 $\mu$m sind und die Kristallite eine Kristallitgröße von 0,5 bis 20 nm aufweisen.

3. Wachstumsinhibierter Hydroxylapatit nach Anspruch 1 oder 2, **dadurch gekennzeichnet, dass** die vorstrukturierten Kollagentemplate aus vorstrukturiertem, denaturiertem Kollagen, vorstrukturierten Kollagenmolekülen, vorstrukturierten Kollagenmikrofibrillen, vorstrukturierten Kollagenfibrillen, vorstrukturierten Kollagenanaloga und/oder vorstrukturierten Kollagenfragmenten und/oder vorstrukturierten Kollagenderivaten und die vorstrukturierten Kollagentemplate eine Länge von weniger als 1 $\mu$m und einen Durchmesser zwischen 1,5 und 100 nm aufweisen.

4. Wachstumsinhibierter Hydroxylapatit nach einem der vorgenannten Ansprüche, **dadurch gekennzeichnet, dass** der wachstumsinhibierte Hydroxylapatit in Agglomeraten von Gelatinemolekülen enthalten ist, an denen epitaktisch HAP-Kristallite mit einer Kristallitgröße unterhalb ihres kritischen Keimradius formiert sind, wobei die Gelatinemoleküle einen Durchmesser zwischen 1,5 und 20 nm, und die HAP-Kristallite eine Korngröße von 1 bis 5 nm aufweisen.

5. Verfahren zur Herstellung von wachstumsinhibiertem HAP mit den Schritten

a) Mineralisation von vorstrukturierten Kollagentemplaten in übersättigten Ca- und Phosphationen aufweisenden Lösungen, wobei die vorstrukturierten Kollagentemplate diffusions- bzw. migrationsfähig sind, so dass eptaktisch HAP-Kristallite an den Kollagentemplaten aufwachsen und sich die mit HAP-Kristalliten aufgewachsenen Kollagentemplate aufgrund deren Diffusions- bzw. Migrationsfähigkeit agglomerieren,

b) Abtrennen der Agglomerate,

wobei als vorstrukturierte Kollagentemplate mit Zuckermolekülen und/oder nichtkollagenen Proteinen vorstrukturierte Kollagentemplate verwendet werden.

6. Verfahren nach Anspruch 5, **dadurch gekennzeichnet, dass** als vorstrukturierte Kollagentemplate vorstrukturiertes, denaturiertes Kollagen, vorstrukturierte Kollagenmoleküle, vorstrukturierte Kollagenmikrofibrillen, vorstrukturierte Kollagenanaloga, vorstrukturierte Kollagenderivate und/oder vorstrukturierte Kollagenfragmente mit einer Länge < 500nm und einem Durchmesser von 1,5 bis 20 nm verwendet werden.

7. Verfahren nach einem der Ansprüche 5 bis 6, **dadurch gekennzeichnet, dass** die Mineralisation stromlos oder elektrochemisch erfolgt.

8. Verfahren nach einem der Ansprüche 5 bis 7, **dadurch gekennzeichnet, dass** die Mineralisation in Teilschritten, beginnend mit einer calciumionenhaltigen, aber phosphatfreien Elektrolytlösung erfolgt.

9. Wachstumsinhibierter Hydroxylapatit nach einem der Ansprüche 1 bis 4 zur Verwendung als Implantatmaterial oder zur Beschichtung von Implantaten.

**Claims**

1. A growth-inhibited hydroxyapatite contained in agglomerates of pre-structured collagen templates the fibrillogenesis of which has been inhibited, onto which hydroxyapatite crystallites with a crystallite size below their critical nucleus radius have been formed epitaxially, and which is unstable in physiological electrolyte solutions and is dissolved therein, wherein the pre-structuring can be imparted by means of sugar molecules or non-collagen proteins.

2. The growth-inhibited hydroxyapatite as claimed in claim 1, **characterized in that** the agglomerates are smaller than 1 $\mu$m and the crystallites have a crystallite size of 0.5 nm to 20 nm.

3. The growth-inhibited hydroxyapatite as claimed in claim 1 or claim 2, **characterized in that** the pre-structured collagen templates are formed from pre-structured denatured collagen, pre-structured collagen molecules, pre-structured collagen microfibrils, pre-structured collagen fibrils, pre-structured collagen analogues and/or pre-structured collagen fragments and/or pre-structured collagen derivatives, and the pre-structured collagen templates have a length of less than 1 $\mu$m and a diameter in the range 1.5 to 100 nm.

4. The growth-inhibited hydroxyapatite as claimed in one of the preceding claims, **characterized in that** the growth-inhibited hydroxyapatite is contained in agglomerates of gelatine molecules on which HAP crystallites with a crystallite size of less than their critical nucleus radius have been formed epitaxially, wherein the gelatine molecules have a diameter in the range 1.5 to 20 nm and the HAP crystallites have a granulometry of 1 to 5 nm.

5. A method for the manufacture of growth-inhibited HAP, having the steps:

a) mineralization of pre-structured collagen templates in supersaturated solutions comprising Ca ions and phosphate ions, wherein the pre-structured collagen templates are capable of diffusing or migrating, so that HAP crystallites grow epitaxially on the collagen templates and the collagen templates with the grown-on HAP crystallites agglomerate on account of their diffusion or migration capability,
b) separation of the agglomerates,
wherein collagen templates pre-structured with sugar molecules and/or non-collagen proteins are used as the pre-structured collagen template.

6. The method as claimed in claim 5, **characterized in that** pre-structured denatured collagen, pre-structured denatured collagen molecules, pre-structured collagen microfibrils, pre-structured collagen analogues, pre-structured collagen derivatives and/or pre-structured collagen fragments with a length of < 500 nm and a diameter of 1.5 to 20 nm is/are used as the pre-structured collagen templates.

7. The method as claimed in claim 5 or claim 6, **characterized in that** the mineralization is carried out without current or electrochemically.

8. The method as claimed in one of claims 5 to 7, **characterized in that** the mineralization is carried out in sub-steps beginning with a calcium ion-containing but phosphate-free electrolyte solution.

9. The growth-inhibited hydroxyapatite as claimed in one of claims 1 to 4, for use as an implant material or for coating implants.

**Revendications**

1. Hydroxyapatite à croissance inhibée, laquelle est contenue dans des agglomérés de matrices de collagène préstructurées dont la fibrillogénèse est empêchée et qui sont pourvues épitactiquement de cristallites d'hydroxyapatite dont la taille de cristallite est inférieure à leur rayon de germination critique, et laquelle est instable dans des solutions d'électrolytes physiologiques alors qu'elle s'y dissout, ladite préstructuration pouvant être obtenue avec des molécules de sucre ou des protéines autres que le collagène.

2. Hydroxyapatite à croissance inhibée selon la revendication 1, **caractérisée en ce que** lesdits agglomérés sont de dimension inférieure à 1 $\mu$m et les cristallites présentent une taille de cristallite comprise entre 0,5 et 20 nm.

3. Hydroxyapatite à croissance inhibée selon les revendications 1 ou 2, **caractérisée en ce que** lesdites matrices de collagène préstructurées sont constituées de collagène dénaturé préstructuré, de molécules de collagène préstructurées, de microfibrilles de collagène préstructurées, de fibrilles de collagène préstructurées, d'analogues de collagène préstructurés et/ou de fragments de collagène préstructurés et/ou de dérivés de collagène préstructurés, alors que les matrices de collagène préstructurées présentent une longueur inférieure à 1 $\mu$m et un diamètre compris entre 1,5 et 100 nm.

4. Hydroxyapatite à croissance inhibée selon l'une des revendications précédentes, **caractérisée en ce que** l'hydroxyapatite à croissance inhibée est contenue dans des agglomérés de molécules de gélatine pourvues épitactiquement de cristallites HAP dont la taille de cristallite est inférieure à leur rayon de germination critique, les molécules de gélatine présentant un diamètre compris entre 1,5 et 20 nm et les cristallites HAP une granulométrie comprise entre 1 et 5 nm.

5. Procédé de préparation de HAP à croissance inhibée, comportant les étapes suivantes

a) minéralisation de matrices de collagène préstructurées dans des solutions sursaturées comportant des ions de Ca et de phosphate, lesdites matrices de collagène préstructurées étant capables de diffusion et/ou de migration, pour faire en sorte qu'une croissance épitactique de cristallites HAP ait lieu sur lesdites matrices de collagène et que les matrices de collagène, pourvues de cristallites HAP suite ladite croissance, subissent une agglomération en raison de leur capacité de diffusion et/ou de migration,

b) séparation des agglomérés,

les matrices de collagène préstructurées mises en oeuvre étant des matrices de collagène préstructurées au moyen de molécules de sucre et/ou des protéines autres que le collagène.

6. Procédé selon la revendication 5, **caractérisé en ce que** l'on utilise en tant que matrices de collagène préstructurées du collagène dénaturé préstructuré, des molécules de collagène préstructurées, des microfibrilles de collagène préstructurées, des analogues de collagène préstructurés et/ou des fragments de collagène préstructurés, présentant une longueur < 500 nm et un diamètre compris entre 1,5 et 20 nm.

7. Procédé selon l'une des revendications 5 à 6, **caractérisé en ce que** ladite minéralisation est effectuée de manière non-électrolytique ou de manière électrochimique.

8. Procédé selon l'une des revendications 5 à 7, **caractérisé en ce que** ladite minéralisation est effectuée en étapes partielles, en commençant par une solution d'électrolytes contenant des ions de calcium mais dépourvue de phosphate.

9. Hydroxyapatite à croissance inhibée selon l'une des revendications 1 à 4, destinée à être utilisée comme matériau d'implant ou pour revêtir des implants.

Fig.1b

Fig.1a

Fig. 2

Fig. 3

Fig. 4

## IN DER BESCHREIBUNG AUFGEFÜHRTE DOKUMENTE

**In der Beschreibung aufgeführte Patentdokumente**

- DE 102004058893 A1
- WO 2007009477 A

**In der Beschreibung aufgeführte Nicht-Patentliteratur**

- **WINTERMANTEL, E. ; HA, S.-W.** Medizintechnik - Life Science Engineering. Springer Verlag, 2008
- Kapitel Forschung aktuell. Knochen auf den Zahn gefühlt. 2005, vol. 1, 10-1
- **TADIC D ; EPPLE M.** A thorough physicochemical characterisation of 14 calcium phosphate-based bone substitution materials in comparison to natural bone. *Biomaterials,* 2004, vol. 25 (6), 987-94
- **MURUGAN R ; RAMAKRISHNA S.** Development of nanocomposites for bone grafting. *Comp Sci Tech,* 2005, vol. 65, 2385-406
- **DETSCH RA ; MAYR HB ; SEITZ DB ; ZIEGLER G.** Is hydroxyapatite ceramic included in the bone remodelling process? An in vitro study of resorption and formation processes. *Key Engineering Materials,* 2008, 361-363, 1123-1126
- **MULLER P ; BULNHEIM U ; DIENER A ; LUTHEN F ; TELLER M ; KLINKENBERG ED ; NEUMANN HG ; NEBE B ; LIEBOLD A ; STEINHOFF G.** Calcium phosphate surfaces promote osteogenic differentiation of mesenchymal stem cells. *J Cell Mol Med,* 2008, vol. 12 (1), 281-91
- **DETSCH R ; MAYR H ; ZIEGLER G.** Formation of osteoclast-like cells on HA and TCP ceramics. *Acta Biomater,* 2008, vol. 4 (1), 139-48
- **MALAFAYA PB ; REIS RL.** *Acta Biomater,* 2009, vol. 5, 644-60